# EUROPEAN PATENT SPECIFICATION

(11) **EP 1 537 075 B1**
(45) Date of publication and mention of the grant of the patent: **01.07.2009**
(21) Application number: 03793605.1
(22) Date of filing: 04.09.2003
(51) Int. Cl.: C07C 275/26, C07C 275/28, C07D 257/04, C07D 207/00, A61K 31/17, A61K 31/41, A61P 19/00, A61P 43/00

(54) **DIARYLUREA DERIVATIVES AND THEIR USE AS CHLORIDE CHANNEL BLOCKERS**
DIARYLHARNSTOFFDERIVATE UND DEREN VERWENDUNG ALS CHLORIDKANALBLOCKER
DERIVES DE DIARYLUREE ET LEUR UTILISATION COMME BLOQUEURS DE CANAUX CHLORURE

(30) Priority: 05.09.2002 DK 200201310; 05.09.2002 DK 200201306
(43) Date of publication of application: 08.06.2005
(73) Proprietor: NeuroSearch A/S, 2750 Ballerup (DK)
(72) Inventor: DAHL, Bjarne, H., 3540 Lynge (DK); CHRISTOPHERSEN, Palle, DK-2750 Ballerup (DK); ENGSIG, Michael, Thyrring, DK-2800 Kongens Lyngby (DK); KARSDAL, Morten, Asser, DK-2100 Kobenhavn (DK); FOGED, Niels, T kker, DK-3670 Vekso (DK); JENSEN, Flemming, Reissig, DK-2100 Kobenhavn (DK)
(74) Representative: Abildgren, Michael Padkjaer
(86) International application number: PCT/DK2003/000575
(87) International publication number: WO 2004/022529

(56) References cited:
- WO-A-00/24707
- WO-A-01/12188
- WO-A-01/76530
- WO-A-02/39987
- WO-A-94/22807
- WO-A-97/45111
- WO-A-97/45400
- WO-A-98/47879
- WO-A-02/064128
- WO-A-02/092576
- WO-A-03/000245

## Description

### TECHNICAL FIELD

The present invention relates to novel diarylurea derivatives useful as chloride channel blockers.

In other aspects the invention relates to the use of these compounds in a method for therapy, such as for the treatment of bone metabolic diseases, diseases responsive to modulation of the mast cell or basophil activity, diseases responsive to inhibition of angiogenesis, or sickle cell anaemia, and to pharmaceutical compositions comprising the compounds of the invention.

### BACKGROUND ART

Chloride channels serve a wide variety of specific cellular functions and contribute to the normal function of i.a. skeletal and smooth muscle cells. Chloride channels are probably found in every cell, from bacteria to mammals. Their physiological tasks range from cell volume regulation to stabilization of the membrane potential, transepithelial or transcellular transport and acidification of intracellular organelles.

WO 97/45400, WO 98/47879, WO 00/20378 and WO 00/24707 (all NeuroSearch A/S) describe compounds, such as substituted phenyl derivatives, active as chloride channel blockers.

WO 02/39987 describes the use of malaria parasite anion channel blockers for the treatment of malaria. WO 02/064128 describes the use of a compound that modulates the association of caspase-9 to Apaf-1 for the treatment of diseases characterized by excessive or insufficient cell death. WO 97/45111 describes phenyl derivatives useful as blockers of chloride channels. WO 01/12188 describes 3(5)-ureido-pyrazole derivatives and their use as antitumor agents.

However, there is a strong interest in the provision of more effective and selective compounds with fewer side effects for the treatment of patients with an osteoclast related bone disease, such as osteoporosis.

Also, there is a strong interest in the provision of more effective and selective compounds with fewer side effects for the treatment of patients with diseases responsive to modulation of the mast cell or basophil activity, diseases responsive to inhibition of angiogenesis, or sickle cell anaemia.

### SUMMARY OF THE INVENTION

It is an object of the invention to provide novel compounds which act as chloride channel blockers.

A further object of the invention is the provision of compounds with a better selectivity. A still further object is the provision of compounds with a better potency.

A further object of the invention is the provision of compounds that act on cell or tissue specific chloride channels, such as such as chloride channels of osteoclasts. A still further object of the invention is the provision of compounds that act on cell or tissue specific chloride channels, such as such as chloride channels of mast cells or basophils. A further object is the provision of compounds that act on specific groups or subtypes of chloride channels.

A still further object is the provision of compound with more optimal pharmacodynamic properties such as kinetic behaviour, bioavailability, solubility and efficacy.

In its first aspect, the invention provides a compound of general formula I, 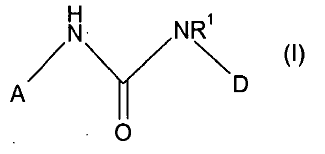 or a pharmaceutically acceptable salt thereof, wherein A, R¹, and D are as defined below.

In its second aspect, the invention provides a pharmaceutical composition, comprising a therapeutically effective amount of a compound of the invention, or a pharmaceutically acceptable salt thereof, together with at least one pharmaceutically acceptable carrier, excipient or diluent.

In a further aspect, the invention provides the use of a compound of the invention, or a pharmaceutically acceptable salt thereof, for the manufacture of a pharmaceutical composition for the treatment, prevention or alleviation of a disease or a disorder or a condition of a mammal, including a human, which disease, disorder or condition is responsive to the blockade of chloride channels.

Other objects of the invention will be apparent to the person skilled in the art from the following detailed description and examples.

### DETAILED DISCLOSURE OF THE INVENTION

### Diarylurea derivatives

In its first aspect, the invention provides a compound of general formula I, 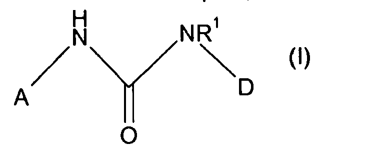 or a pharmaceutically acceptable salt thereof, wherein
A represents a ring system selected from the group consisting of:
pyridyl, thienyl, thiazolyl, indolyl, pyrazolyl and oxo-pyrrolidinyl;
which ring system is optionally substituted with one or more substituents independently selected from the group consisting of:
halo, trifluoromethyl, nitro, alkyl, alkoxy, and phenyl; and
R¹ represents -H; and
D represents 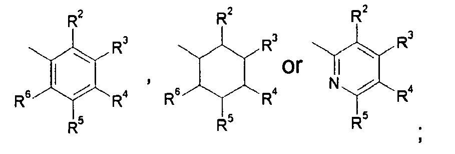 wherein
one of R², R³, and R⁴ is
tetrazolyl; and
R⁵, R⁶ and the remaining one or two of R², R³ and R⁴ independently of each other represent:
o hydrogen, halo, trifluoromethyl,
o -CH=CH-COOR^{b}, -CH₂-CH₂-COOR^{b},
o -CO-NR^{b}-CH₂-COOR^{c}; -CO-NR^{b}R^{c},
o -CH=CH-CO-NR^{b}R^{c}; -CH₂-CH₂-CO-NR^{b}R^{c},
o piperidylcarbonyl,
o -NH-CO-R^{d} or -NH-CO-NH-R^{d};
   wherein R^{d} is phenyl optionally substituted with one or more substituents independently selected from halo or trifluoromethyl; or
o phenyl optionally substituted with
   -SO₂-NR^{b}R^{c}, -CO-NR^{b}R^{c}, -CO-NR^{b}-CH₂-COOR^{c}, or piperidylcarbonyl;
wherein R^{b} and R^{c} independently are hydrogen or alkyl.

In one embodiment of the compound of general formula I, one of R², R³ and R⁴ represents tetrazolyl, such as 1*H*-tetrazol-5-yl. In a special embodiment, R² represents tetrazolyl, such as 1*H*-tetrazol-5-yl. In a further special embodiment, R³ represents tetrazolyl, such as 1*H*-tetrazol-5-yl. In a further special embodiment, R⁴ represents tetrazolyl, such as 1*H*-tetrazol-5-yl. In a further embodiment of the compound of general formula I, R² represents halo, such as bromo. In a further embodiment of the compound of general formula I, R² and R³ both represent halo, such as bromo.

In a further embodiment of the compound of general formula I, A is selected from the group consisting of: 1*H*-indol-2-yl, pyridin-2-yl, pyridin-3-yl, pyridin-4-yl, thiophen-2-yl, thiazol-2-yl, thiazol-3-yl, 2*H*-1λ⁴-thiazol-2-yl, 3,6-dichloro-pyridin-4-yl, 2,6-dichloro-pyridin-4-yl, 5-chloro-pyridin-2-yl, 2-chloro-pyridin-3-yl, 5-phenyl-2*H-*pyrazol-3-yl, and 5-oxo-1-phenyl-pyrrolidin-3-yl.

In one embodiment of D, R⁵ represents halo, such as chloro, bromo or iodo, and R³, R⁴, and R⁶ each represent hydrogen.

In a second embodiment of D, R⁴ represents halo, such as bromo or chloro, and R³, R⁵, and R⁶ each represent hydrogen.

In a further embodiment of D, R³, R⁴, R⁵, and R⁶ each represent hydrogen.

In a further embodiment of D, R⁴ and R⁶ represent halo, such as bromo or chloro, and R³ and R⁵ represent hydrogen.

In a further embodiment of D, R² represents -COOH or -COOCH₃.

In a further embodiment of D, R⁴ represents phenyl, and R³, R⁵, and R⁶ each represent hydrogen.

In a further embodiment of D, R⁵ represents phenyl, and R³ R⁴, and R⁶ each represent hydrogen.

In a further embodiment of D, R² represents -COOH; R⁴ and R⁶ represent halo, such as bromo or chloro, and R³ and R⁵ represent hydrogen.

In a special embodiment, D represents 2-carboxyphenyl, 2-carboxy-4-bromophenyl, 2-carboxy-4-chlorophenyl, 2-carboxy-4-phenylphenyl, 2-carboxy-4,6-dichlorophenyl, 2-carboxy-5-chlorophenyl, 2-carboxy-5-iodophenyl, 2-carboxy-5-phenylphenyl, 2-carboxycyclohexyl, 3-carboxypyridin-2-yl, 3-carboxy-5-bromopyridin-2-yl, 2-methoxycarbonyl-5-chlorophenyl, or 2-methoxycarbonyl-4-bromorophenyl.

In a still further embodiment of the compound of general formula I,
A represents a ring system selected from the group consisting of:
pyridyl;
which ring system is optionally substituted with one or more substituents independently selected from the group consisting of:
halo, trifluoromethyl, nitro, alkyl, and alkoxy;
R¹ represents -H; and
D represents 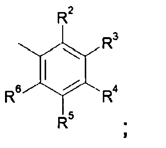 wherein
R² represents tetrazolyl;
R³, R⁴, R⁵, and R⁶ independently of each other represent:
o hydrogen, halo, trifluoromethyl; or
o phenyl substituted with
   -SO₂-NR^{b}R^{c}, -CO-NR^{b}R^{c}, -CO-NR^{b}-CH₂-COOR^{c}, or piperidylcarbonyl;
wherein R^{b} and R^{c} independently are hydrogen or alkyl, such as methyl.

In a special embodiment, A is selected from 2,6-dichloro-pyridin-4-yl, and pyridin-3-yl; and D is selected from 3-chloro-6-(1*H*-tetrazol-5-yl)phenyl, 4-bromo-2-1*H-*tetrazol-5-yl)phenyl, and 4'-(*N,N-*dimethyl-1-carbonyl)-2-(1*H*-tetrazol-5-yl)-biphenyl-4-yl.

In a special embodiment the compound of the invention is
*N*-(2,6-Dichlooro-pyridin-4-yl)-*N'*-[3-chloro-6-(1*H*-tetrazol-5-yl)-phenyl] urea;
*N*-(2,6-Dichloro-pyridin-4-yl)-*N*'-[4'-(*N*",*N*"-dimethyl-1-carbonyl)-2-(1*H*-tetrazol-5-yl)-biphenyl-4-yl] urea;
*N*-(2,6-Dichloro-pyridin-4-yl)-*N*'-[4-bromo-2-(1*H*-tetrazol-5-yl)-phenyl] urea;
*N*-[5-Chloro-2-(1*H*-tetrazol-5-yl)-phenyl]-*N*'-(pyridin-3-yl) urea;
*N*-[4-Bromo-2-(1*H*-tetrazol-5-yl)-phenyl]-*N*'-(pyridin-3-yl) urea;
*N*-[2,4-Dibromo-6-(1*H*-tetrazol-5-yl)-phenyl]-*N*'-(2,6-dichloro-pyridin-4-yl) urea;
or a pharmaceutically acceptable salt thereof.

Any combination of two or more of the embodiments described herein is considered within the scope of the present invention.

### Definition of Substituents

In the context of this invention halo represents fluoro, chloro, bromo or iodo.

Alkyl means a straight chain or branched chain of one to six carbon atoms, including but not limited to, methyl, ethyl, propyl, isopropyl, butyl, isobutyl, t-butyl, pentyl, and hexyl; methyl, ethyl, propyl and isopropyl are preferred groups.

Alkoxy is O-alkyl, wherein alkyl is as defined above.

Amino is NH₂ or NH-alkyl or N-(alkyl)₂, wherein alkyl is as defined above.

### Steric Isomers

The chemical compounds of the present invention may exist in (+) and (-) forms as well as in racemic forms. The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or l- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### Pharmaceutically Acceptable Salts

The chemical compound of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Examples of pharmaceutically acceptable cationic salts of a chemical compound of the invention include, without limitation, the sodium, the potassium, the calcium, the magnesium, the zinc, the aluminium, the lithium, the choline, the lysine, and the ammonium salt, and the like, of a chemical compound of the invention containing an anionic group. Such cationic salts may be formed by procedures well known and described in the art.

In the context of this invention the "onium salts" of N-containing compounds are also contemplated as pharmaceutically acceptable salts (aza-onium salts). Preferred aza-onium salts include the alkyl-onium salts, in particular the methyl- and the ethyl-onium salts; the cycloalkyl-onium salts, in particular the cyclopropyl-onium salts; and the cycloalkylalkyl-onium salts, in particular the cyclopropyl-methyl-onium salts.

### Methods of Preparation

The compounds of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography.

### Pharmaceutical Compositions

In another aspect the invention provides novel pharmaceutical compositions comprising a therapeutically effective amount of a compound of the invention.

While a compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising a compound of the invention, or a pharmaceutically acceptable salt or derivative thereof, together with one or more pharmaceutically acceptable carriers therefore, and, optionally, other therapeutic and/or prophylactic ingredients, knew and used in the art. The carrier(s) must be "acceptable" in the sense of being compatible with the other ingredients of the formulation and not harmful to the recipient thereof. In a further embodiment, the invention provides pharmaceutical compositions comprising more than one compound of the invention, such as two different compounds of the invention.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid, which is in a mixture with the finely-divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, intended for conversion shortly before use to liquid form preparations for oral administration. Such liquid forms include solutions, suspensions, and emulsions. In addition to the active component such preparations may comprise colorants, flavours, stabilisers, buffers, artificial and natural sweeteners, dispersants, thickeners, solubilizing agents.

For topical administration to the epidermis the chemical compound of the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

A therapeutically effective dose refers to that amount of active ingredient, which ameliorates the symptoms or condition. Therapeutic efficacy and toxicity, e.g. ED₅₀ and LD₅₀, may be determined by standard pharmacological procedures in cell cultures or experimental animals. The dose ratio between therapeutic and toxic effects is the therapeutic index and may be expressed by the ratio LD₅₀/ED₅₀. Pharmaceutical compositions exhibiting large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from 0.1 to 500 mg of active ingredient per individual dose, preferably of from 1 to 100 mg, most preferred of from 1 to 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be 10 mg/kg i.v. and 100 mg/kg p.o. Preferred ranges are from 0.1 µg/kg to 10 mg/kg/day i.v., and from 1 µg/kg to 100 mg/kg/day p.o.

### Biological Activity

The compounds of the present invention are useful as blockers of chloride channels, such as chloride channels of osteoclasts. In a further embodiment, the compounds of the present invention are useful as blockers of chloride channels of mast cells and basophils. For measuring the activity of the compounds, various in vitro and in vivo methods, such as various osteoclast inhibition assays known in the art can be used.

### Methods of Therapy

Compounds that are active as chloride channels blockers are likely to be useful in the treatment of a number of diseases, disorders and conditions, including bone metabolic diseases. Further, compounds that are active as chloride channel blockers are likely to be useful in the treatment of diseases responsive to modulation of the mast cell or basophil activity, diseases responsive to inhibition of angiogenesis, or sickle cell anaemia.

Thus in a further aspect, the compounds of the invention are considered useful for the treatment, prevention or alleviation of a disease, disorder or condition responsive to the blockade of chloride channels.

In a special embodiment, the disease or a disorder or a condition is a bone metabolic disease, such as an osteoclast related bone disease. In a further embodiment, the disease or a disorder or a condition is an osteoclast related bone disease, such as osteoporosis, postmenopausal osteoporosis, secondary osteoporosis, osteolytic breast cancer bone metastasis, osteolytic cancer invation, and Paget's disease of bone.

In a further special embodiment, the disease or a disorder or a condition is responsive to modulation of the mast cell or basophil activity. In a still further embodiment, the disease or a disorder or a condition is responsive to modulation of mast cell or basophil production or secretion of histamine, neutral proteases or tryptases (such as chymotryptases and carboxypeptidases), leukotrienes (such as LTC4, and LTB4), prostaglandins (such as PGD2), TXA2, PAF, or cytokines (such as IL-4 and TNF-α). In a further embodiment, the disorder or disease that is responsive to modulation of the mast cell or basophil activity is a disorder or disease that is responsive to modulation of mast cell or basophil production or secretion of histamine. In a still further embodiment, the disorder or disease that is responsive to modulation of the mast cell or basophil activity is allergic bronchopulmonary aspergillosis (ABPA), allergic rhinitis, allergic skin disease, allergic skin reaction, drug induced allergic skin reaction, anaphylaxis, asthma, atherosclerosis, atopic dermatitis (AD), bronchial asthma, cancer, chronic obstructive pulmonary disease (COPD), Chrohn's disease, contact dermatitis, dilated cardiomyopathy, fatal asthma, graft rejection, hypersensitivity pneumonitis, ischemic hearth disease, pulmonary fibrosis, rheumatoid arthritis, systemic sclerosis, urticaria, or uveoretinitis. In a special embodiment, the disorder or disease that is responsive to modulation of the mast cell or basophil activity is allergic bronchopulmonary aspergillosis (ABPA), allergic rhinitis, allergic skin disease, allergic skin reaction, drug induced allergic skin reaction, asthma, bronchial asthma, fatal asthma or chronic obstructive pulmonary disease (COPD). In a further special embodiment, the disorder or disease is asthma, bronchial asthma, fatal asthma or chronic obstructive pulmonary disease (COPD). In a further special embodiment, the disorder or disease is COPD. In a still further special embodiment, the disorder or disease is asthma.

In a further special embodiment, the disease or a disorder or a condition is responsive to inhibition of angiogenesis. In a special embodiment, the diseases, disorders or conditions that are responsive to inhibition of angiogenesis are selected from:
- diseases, disorders or conditions that involve the proliferation of tumor cells, such as cancer, prostate cancer, lung cancer, breast cancer, bladder cancer, renal cancer, colon cancer, gastric cancer, pancreatic cancer, ovarian cancer, melanoma, hepatoma, sarcoma and lymphoma;
- ophthalmic angiogenesis related diseases, disorders or conditions, such as exudative macular degeneration, age-related mucular degeneration (AMD), retinopathy, diabetic retinopathy, proliferative diabetic retinopathy, diabetic macular edema (DME), ischemic retinopathy (e.g. retinal vain or artery occlusion), retinopathy of prematurity, neovascular glaucoma, and corneal neovascularization; and
- rheumatoid arthritis, and psoriasis.

In a special embodiment, the disease, disorder or condition to be treated is a preneoplastic disease state. In a further embodiment, the treatment is an anti-metastatic treatment. In a still further embodiment, the disease, disorder or condition to be prevented is metastatic cancer. In a further embodiment, the disease, disorder or condition to be prevented or alleviated is DME.

In the context of this invention, "age-related macular degeneration" (AMD) includes dry AMD (non-exudative AMD) and wet AMD (exudative AMD).

In a still further embodiment, the disease, disorder or condition responsive to the blokade of chloride channels is sickle cell anaemia, brain oedema following ischaemia or tumors, diarrhea, hypertension, diuretic hypertension, glaucoma, or ulcers.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge. When administered in combination with compounds known in the art for treatment of the diseases, the dosis regimen may be reduced.

Use of the compounds of the invention may be combined with the use of one or more additional drugs.

Use of the compounds of the invention may be combined with the use of other bone metabolism controlling compounds for the treatment of bone metabolic disease. Such known bone metabolism controlling compounds include bisphophonates such as etidronate, pamidronate, or clodronate optionally combined with calcium; oestrogen-receptor active compounds such as oestrogen i.e. oestradiol and ethyloestradiol, calcitonin, 1,25-dihydroxyvitamine D and metabolites thereof, fluoride, growth hormone, parathyroid hormone, triiodo-thyrosine, collagen degrading enzymes such as protease inhibitors, or cancer therapeutic agents.

Further, use of the compounds of the invention may be combined with the use of one or more additional drugs useful for the treatment, prevention or alleviation of a disease responsive to inhibition of angiogenesis, such as compounds useful for anti-metastatic treatment. Such additional drugs include cytotoxic compounds, antimitotic compounds, and antimetabolites.

Examples of cytotoxic compounds (including cytotoxic alkylating agents) include carmustine (BCNU), fotemustin, temozolomide (temodal), ifosfamide, and cyclofosfamide.

Examples of antimitotic compounds include paclitaxel (taxol) and docetaxel.

An example of antimetabolites includes methotrexat.

Furthermore, the compounds of the invention may be combined or administered in combination with other treatments or therapies. Examples of other treatments or therapies include radiotherapy and surgery.

The treatment of the diseases and disorder can be in chronical or long term treatment as well as a treatment of sudden crisis in the disease and disorder.

### EXAMPLES

The invention is further illustrated with reference to the following examples, which are not intended to be in any way limiting to the scope of the invention as claimed.

### Example 1

### (2-Amino-phenyl)-phosphonic acid diethyl ester

Diethylphosphite (1.5 g, 11 mmol) in liquid ammonia (app. 100 ml) was added potassium tert.-butoxide (1.23 g, 11 mmol), the reaction mixture was stirred for 10 min. before 2-iodoaniline was added. The reaction mixture was irradiated (Hg-lamp) for an hour. Ammonia was evaporated and ammonium chloride was added with some ammonia, ammonia was evaporated off. The residue was added water and extracted with dichloromethane. The title compound was purified by column chromatography. Yield 1.7 g (yellow oil).

### Example 2

### (2-Amino-5-bromo-phenyl)-phosphonic acid diethyl ester

(2-Amino-phenyl)-phosphonic acid diethyl ester (0.23g, 1 mmol) in glacial acetic acid (2 ml) under stirring was added bromine (0.16 g, 1 mmol, 1 eq.) in glacial acetic acid (2 ml) over 20 min. After the reaction was completed, the reaction mixture was poured into a saturated aqueous sodium bicarbonate solution. The title compound was extracted with dichloromethane and purified by column chromatography. Yield 0.23 g

### Example 3

### [(Dimethoxy-phosphoryl)-hydroxy-(2-nitro-phenyl)-methyl)-phosphonic acid dimethyl ester

Trimethyl phosphite (5.7 g, 45.7 mmol) was cooled to -10°C under a argon atmosphere and glacial acetic acid was added (1.44 ml), while keeping the temperature below 0°C, was drop wise added 2-nitrobenzoyl chloride, the reaction mixture was allowed to heat to room temperature and stirred for one hour. At a pressure of 10 mmHg was the reaction mixture heated to 40°C for one hour and cooled to room temperature. Ethyl acetate was added and the solution was washed with 5% sodium bicarbonate (aq.), the organic phase was dried and evaporated, the title compound was purified by column chromatography.

The following compound was made analogously; [(Dimethoxy-phosphoryl)-hydoxy-(3-chloro-6-nitro-phenyl)-methyl]-phosphonic acid dimethyl ester.

### Example 4

### 5-Amino-furan-2-carboxylic acid

5-Nitro-2-furoic acid methyl ester (14.3 g, 84 mmol) in ethyl acetate (110 ml) was added palladium on charcoal (0.75 g, 5%), the solution was stirred at 40°C under a hydrogen atmosphere for 4 hours, the reaction mixture was filtered through celite, the title compound was isolated by evaporation and used as this in the next reaction.

### Example 5

### 4-Amino-1-hydroxy-cyclohexa-2,4-dienecarboxylic acid methyl ester

5-Amino-furan-2-carboxylic acid (the crude product from example 4) was dissolved in benzene (0.5 L) and added acrylonitrile (200 ml), the reaction mixture was heated at reflux, stirred for 16 hours and evaporated, the residue was added toluene and evaporated. The title compound was crystallized from ethyl acetate, and used with out further purification in the next reaction.

### Example 6

### 4-Amino-3-cyano-benzoic acid methyl ester

4-Amino-1-hydroxy-cyclohexa-2,4-dienecarboxylic acid methyl ester (the crude product from example 5) was dissolved in toluene (600 ml) and added boron trifluoride diethyl etherate (20 ml of 40% in diethyl ether), the reaction mixture was added tetrahydrofurane (100 ml) and stirred at 85°C for 30 min. The reaction mixture was cooled to room temperature and poured into ice/water, the mixture was neutralized with sodium hydroxide (1 M) and basified with saturated sodium bicarbonate. The layer was separated and the water phase was extracted with dichloro methane. The organic phases was dried and evaporated. The title compound was isolated from the residue by column chromatography. Yield 3.2 g.

### Example 7

### 4-Amino-3-cyano-benzoic acid

4-Amino-3-cyano-benzoic acid methyl ester (3.2 g, 10 mmol) in tetrahydrofurane/ water (110 ml, 1:1) was added lithium hydroxide hydrate (2.3 g, 30 mmol), the reaction mixture was stirred at room temperature for 20 hours and added hydro chloric acid (1 M) to pH = 3. From the mixture was tetrahydrofurane distilled of and the title compound precipitated out of solution.

### Example 8

### 4-Amino-3-cyano-N-methyl-benzamide

4-Amino-3-cyano-benzoic acid (0.97 g, 6 mmol), benzotriazol-1-yloxytris-(pyrrolidino)-phosphonium hexafluorophosphate (3.3 g, 6.3 mmol) and diisopropylethylamine (6 ml, 12 mmol) in anhydrous dimethyl formamide (25 ml) was added dimethylamine (6 ml of a 2 M in tetrahydrofurane), the reaction mixture was stirred at room temperature overnight. Some of the dimethyl formamide was evaporated off, the crude product was dissolved in ethyl acetate (25 ml), washed with potassium hydrogen sulphate (twice with 0.5 M (25 ml)), sodium hydroxide (twice with 1 M (25 ml)), water (10 ml) and brine (25 ml). The organic layer was dried and evaporated, the title compound was isolated by column chromatography. Yield 0.39 g.

The following compounds were made analogously:
4-Amino-3-cyano-*N*,*N*-dimethyl-benzamide;
(4-Amino-3-cyano-benzoylamino)-acetic acid ethyl ester;
2-Amino-5-(piperidine-1-carbonyl)-benzonitrile;
4-Amino-3-cyano-*N,N*-diethyl-benzamide;
[(4-Amino-3-cyano-benzoyl)-methyl-amino]-acetic acid.

### Example 9

### (2-Amino-benzyl)-phosphonic acid dimethyl ester

[(Dimethoxy-phosphoryl)-hydroxy-(2-nitro-phenyl)-methyl]-phosphonic acid dimethyl ester (4.7 g, 17.7 mmol) in 99% ethyl alcohol (40 ml) was added Tin(II) chloride dihydrate (14.4 g, 64 mmol, 5 eq.), the reaction mixture was heated at 70°C for 20 min. and poured into ice (250 ml), pH was adjusted to 7 with sodium hydroxide (1 M) and ethyl acetate was added, the emulsion was filtered through kieselguhr twice, the organic layer was washed with brine, dried, evaporated and the title compound was isolated as an oil. Yield 2 g.

The following compound was made analogously:
(2-Amino-5-chloro-benzyl)-phosphonic acid dimethyl ester.

### Example 10

### 2-Amino-benzeneboronic acid

2-Nitro-benzeneboronic acid (2.32 g, 14 mmol) in ethyl alcohol (130 ml, 99%) was added palladium on charcoal (0.232 g, 10%), stirred under a hydrogen atmosphere (3 bar) overnight and filtered. The filtrate was evaporated, the title compound was crystallized from methyl alcohol and water. Yield 0.52 g.

### Example 11

### 4-Methyl-phenyl-boronic acid

4-Bromo-toluene (50 g, 0.29 mol) in anhydrous diethyl ether (500 ml) was cooled to - 20°C, while keeping the temperature under - 20°C, was butyl lithium (127 ml of an 2.5 M solution in hexane 0.31 mol) added, after 15 min the solution was allowed to heat to 15°C and stirred at that temperature for an hour, then cooled to - 55°C, while keeping the temperature under - 47°C was tributylborate (110 ml, 94 g, 0.41 mol, 1.4 eq.) added, the reaction mixture was allowed to heat to room temperature and stirred overnight. Hydrochloric acid (450 ml of 1 M) was added. The phases were separated, the water phase was extracted with diethyl ether, the organic phases werer combined and extracted with sodium hydroxide (aq.) (300 ml + 2 times with 100ml of 2 M), the water phases were added concentrated hydrochloric acid (app. 100 ml), the title compound precipitated and was isolated by filtration. Yield 32.6 g

### Example 12

### 4-(Dihydroxyboryl) benzoic acid

4-Methyl-phenyl-boronic acid (32.6 g 0.24 mol) was dissolved in water (900 ml) containing sodium hydroxide (19.2 g 0.48 mol, 2 eq.) and added potassium permanganate (79.6 g, 0.5 mol, 2.1 eq.), the reaction mixture was stirred at room temperature for 72 hours and filtered, the precipitate was washed three times with water (50 ml), the filtrate was added concentrated hydrochloric acid (app. 55 ml) and the title compound precipitated. Yield 34.5 g.

### Example 13

### 4-(Dihydroxyboryl)-(N,N-dimethylbenzamide)

4-(Dihydroxyboryl) benzoic acid (34.4 g, 0.21 mol) was added to thionyl chloride (300 ml), the reaction mixture was heated at reflux overnight and evaporated to dryness, the residue was added dimethylamine (167 ml of a 40% solution in water), the reaction mixture was heated at reflux for 20 min. and filtered while still hot, the filtrate was cooled to room temperature and added concentrated hydrochloric acid and the title compound precipitated. Yield 25.1 g.

The following compounds were made analogously:
4-(Dihydroxyboryl)-benzamide;
[4-(Dihydroxyboryl)-benzoylamino]-acetic aid;
[4-(Dihydroxyboryl)-benzoyl-(*N*-methyl)-amino]-acetic acid.

### Example 14

### 4'-Amino-3'-cyano-biphenyl-4-carboxylic acid dimethylamide

4-(Dihydroxyboryl)-(*N*,*N*-dimethylbenzamide) (1.2 g, 6.2 mmol), 2-amino-5-bromobenzonitrile (1.1 g, 5.6 mmol, 0.9 eq), potassium carbonate (2.3g, 16.9 mmol, 3.3 eq.), dimethoxyethylenglycol (20 ml) and water (10 ml) was mixed nitrogen was bobbled through the mixture, bis(triphenylphosphine)palladium (II) chloride (0.05 g) was added, the reaction mixture was heated at reflux for 40 min, cooled to room temperature, added water (50 ml) and extracted with ethyl acetate (50 ml), the organic phase was washed with water (30 ml) dried, evaporated and the title compound was left as an oil. Yield 1.17 g.

### Example 15

### 3-(4-Amino-3-cyano-phenyl)-acrylic acid methyl ester

2-Amino-5-bromo-benzonitrile (0.59 g 3 mmol), methylacrylate (0.52 g, 6 mmol) and tri-o-tolylphosphine (0.49 mg, 0. 16 mmol) in anhydrous *N*,*N*-dimethyl formamide (5 ml) was added triethylamine (0.44 ml), the mixture was bobbled through with argon and palladium (II) acetate (4.5 mg, 0.02 mmol) and stirred at 120°C for 2.5 hours. Then the reaction was finished, the reaction mixture was cooled to room temperature and added hydrochloric acid (15 ml of 1 M), the title compound was extracted with diethyl ether (four times with 25 ml), the organic phase was dried and evaporated.

The following compound was made analogously:
3-(Amino-3-cyano-phenyl)-acryl *N,N*-dimethyl amide.

### Example 16

### 3-(4-Amino-3-cyano-phenyl)-propionic acid methyl ester

3-(4-Amino-3-cyano-phenyl)-acrylic acid methyl ester (10 g, 49 mmol) and palladium on charcoal (2 g, 10%) in tetrahydrofurane (200 ml) was stirred vigorously under a hydrogen atmosphere for 20 min., the reaction mixture was filtered though celite and the title compound was isolated by filtration. Yield 10 g

The following compound was made analogously:
3-(4-Amino-3-cyano-phenyl)-propionic acid *N*,*N*-dimethyl amide.

### Example 17

### 4'-Amino-3'-(1H-tetrazol-5-yl)-biphenyl-4-carboxylic acid dimethylamide

4'-Amino-3'-cyano-biphenyl-4-carboxylic acid dimethylamide (1.15 g, 4.3 mmol) in toluene (25 ml) was added sodium azide (0.42 g, 6.5 mmol, 1.5 eq.) and triethylammonium chloride (0.9 g, 6.5 mmol, 1.5 eq.), the reaction mixture was stirred at 60°C for 48 hours. The top phase was decanted from the bottom layer, the residue was added water (25 ml), 96% ethyl alcohol (25 ml) and concentrated hydro chloric acid (app. 1 ml) and the title compound precipitated out. Yield 0.79 g.

The following compounds were made analogously:
4'-Amino-3-(1*H*-tetrazol-5-yl)-biphenyl-4-dimethyl-sulfon-amide;
2-Bromo-4-(1*H*-tetrazol-5-yl)-aniline;
2,6-Dibromo-4-(1*H*-tetrazol-5-yl)-aniline;
2-Bromo-5-(1*H*-tetrazol-5-yl)-aniline;
3-Chloro-6-(1*H*-tetrazol-5-yl)-aniline;
4-Bromo-2-(1*H*-tetrazol-5-yl)-aniline;
2,4-Dibromo-6-(1*H*-tetrazol-5-yl)-aniline;
[4'-Amino-3'-(1*H*-tetrazol-5-yl)-biphenyl-4-yl]-carbonyl-piperidin-1-yl;
2,4-Dichloro-6-(tetrazol-5-yl)-aniline;
4-Amino-*N*-methyl-3-(1*H*-tetrazol-5-yl)-benzamide;
[4-Amino-3-(1*H*-tetrazol-5-yl)-benzoylamino]-acetic acid;
3-[4-Amino-3-(1*H*-tetrazol-5-yl)-phenyl]-acrylic acid methyl ester;
3-[4-Amino-3-(1*H*-tetrazol-5-yl)-phenyl]-propionic acid methyl ester;
*N*-[4-Amino-3-(1*H*-tetrazol-5-yl)-phenyl]-malonamic acid;
3-[4-Amino-3-(1*H*-tetrazol-5-yl)-phenyl]-*N*,*N*-dimethyl-acrylamide;
3-[4-amino-3-(1*H*-tetrazol-5-yl)-phenyl]-*N*-methyl-acrylamide;
4-Amino-*N*,*N*-diethyl-3-(1*H*-tetrazol-5-yl)-benzamide;
4-Amino-3-(1*H*-tetrazol-5-yl)-benzoyl-piperidin-1-yl;
4'-Amino-3'-(1*H*-tetrazol-5-yl)-biphenyl-4-carboxylic acid amide;
4-Amino-*N*,*N*-dimethyl-3-(1*H*-tetrazol-5-yl)-benzamide;
[4-Amino-*N*-methyl-3-(1*H*-tetrazol-5-yl)-benzoylamino]-acetic acid;
3-[4-Amino-3-(1*H*-tetrazol-5-yl)-phenyl]-*N*-methyl-propionamide;
*N*-[4-Amino-3-(1*H*-tetrazol-5-yl)-phenyl]-benzamide.

### Example 18

### N-(3,5-Bis-trifluoromethyl-phenyl)-N'-[2-(1H-tetrazol-5-yl)-biphenyl-4-yl-4'-carboxylic acid dimethylamide] urea

2'-Amino-3'-(1*H*-tetrazol-5-yl)-biphenyl-4-carboxylic acid dimethylamide (1g, 3.2 mmol) in toluene (25 ml) was added 3,5-bis-(trifluoromethyl)-phenyl isocyanate (0.88 g, 3.3 mmol, app 1 eq.) and triethylamine (0.36 g, 3.6 mmol, 1.2 eq.), the reaction mixture was stirred overnight, an oil in the bottom of the flask was isolated. The oil was dissolved in 2-propanol (20 ml), the solution was added hydrochloric acid (1 M) until pH = 2-3, the title compound precipitated. Yield 0.86 g. M.p. 222-224°C.

The following compounds were made analogously:
*N*-(2,6-Dichlooro-pyridin-4-yl)-*N*'-[3-chloro-6-(1*H*-tetrazol-5-yl)-phenyl] urea; M.p. 201-203°C;
*N*-(2,6-Dichloro-pyridin-4-yl)-*N*'-[4'-(*N*",*N*"-dimethyl-1-carbonyl)-2-(1*H*-tetrazol-5-yl)-biphenyl-4-yl] urea; M.p. 163.8-164.5°C;
*N*-(2,6-Dichloro-pyridin-4-yl)-*N*'-[4-bromo-2-(1*H*-tetrazol-5-yl)-phenyl] urea; M.p. 231-233°C;
*N*-[5-Chloro-2-(1*H*-tetrazol-5-yl)-phenyl]-*N*'-(pyridin-3-yl) urea; M.p. 214-220°C;
*N*-[4-Bromo-2-(1*H*-tetrazol-5-yl)-phenyl]-*N*'-(pyridin-3-yl) urea; M.p. 193-194°C;
*N*-[2,4-Dibromo-6-(1*H*-tetrazol-5-yl)-phenyl]-*N*'-(2,6-dichloro-pyridin-4-yl) urea; M.p. 202-203°C;

## Claims

1. A chemical compound represented by general formula (I) 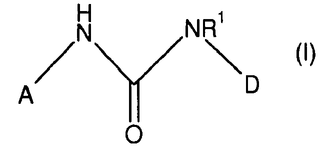 or a pharmaceutically acceptable salt thereof, wherein
A represents a ring system selected from the group consisting of:
pyridyl, thienyl, thiazolyl, indolyl, pyrazolyl and oxo-pyrrolidinyl;
which ring system is optionally substituted with one or more substituents independently selected from the group consisting of:
halo, trifluoromethyl, nitro, alkyl, alkoxy, and phenyl; and
R¹ represents -H; and
D represents 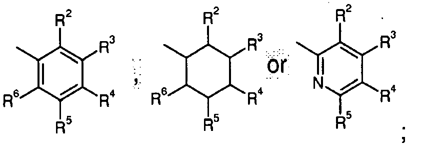 wherein one of R², R³, and R⁴ is tetrazolyl;
and R⁵, R⁶ and the remaining one or two of R², R³ and R⁴ independently of each other represent:
o hydrogen, halo, trifluoromethyl,
o -CH=CH-COOR^{b}, -CH₂-CH₂-COOR^{b},
o -CO-NR^{b}-CH₂-COOR^{c}; -CO-NR^{b}R^{c},
o -CH=CH-CO-NR^{b}R^{c}; -CH₂-CH₂-CO-NR^{b}R^{c},
o piperidylcarbonyl,
o -NH-CO-R^{d} or -NH-CO-NH-R^{d};
wherein R^{d} is phenyl optionally substituted with one or more substituents independently selected from halo or trifluoromethyl; or
o phenyl optionally substituted with
-SO₂-NR^{b}R^{c}, -CO-NR^{b}R^{c}, -CO-NR^{b}-CH₂-COOR^{c}, or piperidylcarbonyl; wherein R^{b} and R^{c} independently are hydrogen or alkyl.

2. The compound of claim 1, wherein
A represents pyridyl;
which pyridyl is optionally substituted with one or more substituents independently selected from the group consisting of:
halo, trifluoromethyl, nitro, alkyl, and alkoxy; and
R¹ represents -H; and
D represents 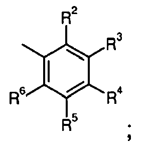 wherein
R² represents tetrazolyl;
R³, R⁴, R⁵, and R⁶ independently of each other represent:
o hydrogen, halo, trifluoromethyl; or
o phenyl substituted with
-SO₂-NR^{b}R^{c}, -CO-NR^{b}R^{c}, -CO-NR^{b}-CH₂-COOR^{c}, or piperidylcarbonyl;
wherein R^{b} and R^{c} independently are hydrogen or alkyl.

3. The compound of claim 1, being
*N*-(2,6-Dichloro-pyridin-4-yl)-*N*'-[3-chloro-6-(1*H*-tetrazol-5-yl)-phenyl] urea;
*N*-(2,6-Dichloro-pyridin-4-yl)-*N*'-[4'-(*N*",*N*"-dimethyl-1-carbonyl)-2-(1*H*-tetrazol-5-yl)-biphenyl-4-yl] urea;
*N*-(2,6-Dichloro-pyridin-4-yl)-*N*'-[4-bromo-2-(1*H*-tetrazol-5-yl)-phenyl] urea;
*N*-[5-Chloro-2-(1*H*-tetrazol-5-yl)-phenyl]-*N*'-(pyridin-3-yl) urea;
*N*-[4-Bromo-2-(1*H*-tetrazol-5-yl)-phenyl]-*N'*-(pyridin-3-yl) urea;
*N*-[2,4-Dibromo-6-(1*H*-tetrazol-5-yl)-phenyl]-*N'*-(2,6-dichloro-pyridin-4-yl) urea;
or a pharmaceutically acceptable salt thereof.

4. A pharmaceutical composition comprising a therapeutically effective amount of a compound according to any of claims 1-3, or a pharmaceutically acceptable salt thereof, together with at least one pharmaceutically acceptable carrier, excipient or diluent.

5. The use of a compound according to any one of claims 1-3, or a pharmaceutically acceptable salt thereof, for the manufacture of a pharmaceutical composition for the treatment, prevention or alleviation of a disease or a disorder or a condition of a mammal, including a human, which disease, disorder or condition is responsive to the blockade of chloride channels.

6. The use according to claim 5, wherein the disease, disorder or condition responsive to the blockade of chloride channels is a bone metabolic disease or an osteoclast related bone disease.

7. The use according to claim 5, wherein the disease, disorder or condition responsive to the blockade of chloride channels is osteoporosis, postmenopausal osteoporosis, secondary osteoporosis, osteolytic breast cancer bone metastasis, osteolytic cancer invation, Paget's disease of bone.

8. The use according to claim 5, wherein the disease, disorder or condition responsive to the blockade of chloride channels is a disease, disorder or condition responsive to the mast cell or basophil activity, or to inhibition of angiogenesis.

9. The use according to claim 5, wherein the disease, disorder or condition responsive to the blockade of chloride channels is allergic bronchopulmonary aspergillosis (ABPA), allergic rhinitis, allergic skin disease, allergic skin reaction, drug induced allergic skin reaction, anaphylaxis, asthma, atherosclerosis, atopic dermatitis (AD), bronchial asthma, cancer, chronic obstructive pulmonary disease (COPD), Chrohn's disease, contact dermatitis, dilated cardiomyopathy, fatal asthma, graft rejection, hypersensitivity pneumonitis, ischemic hearth disease, pulmonary fibrosis, rheumatoid arthritis, systemic sclerosis, urticaria, uveoretinitis, cancer, metastatic cancer, prostate cancer, lung cancer, breast cancer, bladder cancer, renal cancer, colon cancer, gastric cancer, pancreatic cancer, ovarian cancer, melanoma, hepatoma, sarcoma, lymphoma, exudative macular degeneration, age-related mucular degeneration (AMD), retinopathy, diabetic retinopathy, proliferative diabetic retinopathy, ischemic retinopathy (e.g. retinal vain or artery occlusion), retinopathy of prematurity, neovascular glaucoma, corneal neovascularization, rheumatoid arthritis, psoriasis, sickle cell anaemia, brain oedema following ischaemia or tumors, diarrhea, hypertension, diuretic hypertension, glaucoma, or ulcers.

## Patentansprüche

1. Chemische Verbindung, die durch die allgemeine Formel (I) wiedergegeben wird 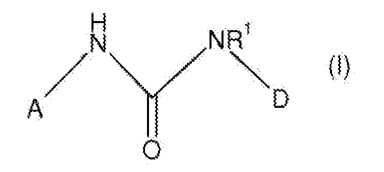 oder ein pharmazeutisch verträgliches Salz davon, wobei
A ein Ringsystem bedeutet, das ausgewählt ist aus der Gruppe bestehend aus:
Pyridyl, Thienyl, Thiazolyl, Indolyl, Pyrazolyl und Oxo-pyrrolidinyl;
wobei dieses Ringsystem gegebenenfalls mit einem oder mehreren Substituenten substituiert ist, die unabhängig voneinander ausgewählt sind aus der Gruppe bestehend aus:
Halogen, Trifluormethyl, Nitro, Alkyl, Alkoxy und Phenyl; und
R¹ -H bedeutet; und
D 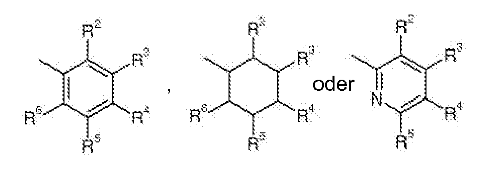 bedeutet;
wobei eines von R², R³ und R⁴ Tetrazolyl ist;
und R⁵, R⁶ und die übrigen ein oder zwei von R², R³ und R⁴ unabhängig voneinander bedeuten:
o Wasserstoff, Halogen, Trifluormethyl,
o -CH=CH-COOR^{b}, -CH₂-CH₂-COOR^{b},
o -CO-NR^{b}-CH₂-COOR^{c}; -CO-NR^{b}R^{c},
o -CH=CH-CO-NR^{b}R^{c}; -CH₂-CH₂-CO-NR^{b}R^{c},
o Piperidylcarbonyl,
o -NH-CO-R^{d} oder -NH-CO-NH-R^{d};
wobei R^{d} Phenyl ist, das gegebenenfalls mit einem oder mehreren Substituenten substituiert ist, die unabhängig voneinander ausgewählt sind aus Halogen oder Trifluormethyl; oder
o Phenyl, das gegebenenfalls substituiert ist mit
-SO₂-NR^{b}R^{c}, -CO-NR^{b}R^{c}, -CO-NR^{b}-CH₂-COOR^{c} oder Piperidylcarbonyl;
wobei R^{b} und R^{c} unabhängig voneinander Wasserstoff oder Alkyl sind.

2. Verbindung nach Anspruch 1, wobei
A Pyridyl bedeutet;
wobei dieses Pyridyl gegebenenfalls mit einem oder mehreren Substituenten substituiert ist, die unabhängig voneinander ausgewählt sind aus der Gruppe bestehend aus:
Halogen, Trifluormethyl, Nitro, Alkyl und Alkoxy; und
R¹ -H bedeutet; und
D 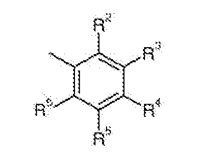 bedeutet;
wobei
R² Tetrazolyl bedeutet;
R³, R⁴, R⁵ und R⁶ unabhängig voneinander bedeuten:
o Wasserstoff, Halogen, Trifluormethyl; oder
o Phenyl, das substituiert ist mit
-SO₂-NR^{b}R^{c}, -CO-NR^{b}R^{c}, -CO-NR^{b}-CH₂-COOR^{c} oder Piperidylcarbonyl;
wobei R^{b} und R^{c} unabhängig voneinander Wasserstoff oder Alkyl sind.

3. Verbindung nach Anspruch 1, bei der es sich um
*N*-(2,6-Dichlor-pyridin-4-yl)-*N*'-[3-chlor-6-(1*H*-tetrazol-5-yl)-phenyl]harnstoff;
*N*-(2,6-Dichlor-pyridin-4-yl)-*N*'-[4'-(*N*'',*N*''-dimethyl-1-carbonyl)-2-(1*H*-tetrazol-5-yl)-biphenyl-4-yl]harnstoff;
*N*-(2,6-Dichlor-pyridin-4-yl)-*N*'-[4-brom-2-(1*H*-tetrazol-5-yl)-phenyl]harnstoff;
*N*-[5-Chlor-2-(1*H*-tetrazol-5-yl)-phenyl]-*N*'-(pyridin-3-yl)harnstoff;
*N*-[4-Brom-2-(1*H*-tetrazol-5-yl)-phenyl]-*N*'-(pyridin-3-yl)harnstoff;
*N*-[2,4-Dibrom-6-(1*H*-tetrazol-5-yl)-phenyl]-*N*'-(2,6-dichlor-pyridin-4-yl)harnstoff handelt;
oder ein pharmazeutisch verträgliches Salz davon.

4. Pharmazeutische Zusammensetzung, umfassend eine therapeutisch wirksame Menge von einer Verbindung nach einem der Ansprüche 1-3, oder einem pharmazeutisch verträglichen Salz davon, zusammen mit wenigstens einem pharmazeutisch verträglichen Träger, Exzipienten oder Verdünnungsmittel.

5. Verwendung einer Verbindung nach einem der Ansprüche 1-3, oder eines pharmazeutisch verträglichen Salzes davon, für die Herstellung einer pharmazeutischen Zusammensetzung für die Behandlung, Verhütung oder Linderung einer Krankheit oder einer Störung oder eines Zustands bzw. Leidens eines Säugers, einschließlich eines Menschen, wobei diese Krankheit, diese Störung oder dieser Zustand bzw. dieses Leiden auf die Blockade von Chloridkanälen anspricht.

6. Verwendung nach Anspruch 5, wobei die Krankheit, die Störung oder der Zustand bzw. das Leiden, die bzw. der bzw. das auf die Blockade von Chloridkanälen anspricht, eine Knochenstoffwechselerkrankung oder eine mit Osteoklasten verbundene Knochenerkrankung ist.

7. Verwendung nach Anspruch 5, wobei es sich bei der Krankheit, der Störung oder dem Zustand bzw. Leiden, die bzw. der bzw. das auf die Blockade von Chloridkanälen anspricht, um Osteoporose, postmenopausale Osteoporose, sekundäre Osteoporose, osteolytische Brustkrebs-Knochenmetastase, osteolytische Krebsinvasion, Morbus Paget (Osteodystrophia deformans) handelt.

8. Verwendung nach Anspruch 5, wobei die Krankheit, die Störung oder der Zustand bzw. das Leiden, die bzw. der bzw. das auf die Blockade von Chloridkanälen anspricht, eine Krankheit, eine Störung oder ein Zustand bzw. ein Leiden ist, die bzw. der bzw. das auf die Mastzell- oder Basophilenaktivität oder auf die Hemmung von Angiogenese anspricht.

9. Verwendung nach Anspruch 5, wobei es sich bei der Krankheit, der Störung oder dem Zustand bzw. Leiden, die bzw. der bzw. das auf die Blockade von Chloridkanälen anspricht, um allergische bronchopulmonale Aspergillose (ABPA), allergische Rhinitis, eine allergische Hauterkrankung, eine allergische Hautreaktion, eine medikamenten- bzw. drogeninduzierte allergische Hautreaktion, Anaphylaxie, Asthma, Atherosklerose, atopische Dermatitis (AD), Asthma bronchiale, Krebs, chronisch-obstruktive Lungenerkrankung (COPD), Morbus Crohn, Kontaktdermatitis, dilatative Kardiomyopathie, fatales Asthma, Transplantatabstoßung, Überempfindlichkeits-Pneumonitis, ischämische Herzerkrankung, Lungenfibrose, rheumatoide Arthritis, systemische Sklerose, Urtikaria, Uveoretinitis, Krebs, metastatischen Krebs, Prostatakrebs, Lungenkrebs, Brustkrebs, Blasenkrebs, Nierenkrebs, Dickdarmkrebs, Magenkrebs, Pankreaskrebs, Eierstockkrebs, Melanom, Hepatom, Sarkom, Lymphom, exsudative Makuladegeneration, altersbedingte Makuladegeneration (AMD), Retinopathie, diabetische Retinopathie, proliferative diabetische Retinopathie, ischämische Retinopathie (z.B. retinalen Venen- oder Arterienverschluss), Frühgeborenenretinopathie, neovaskuläres Glaukom, Hornhautneovaskularisation, rheumatoide Arthritis, Psoriasis, Sichelzellanämie, Hirnödem infolge von Ischämie oder Tumoren, Diarrhö, Hochdruck, diuretischen Hochdruck, Glaukom oder Geschwüre handelt.

## Revendications

1. Composé chimique représenté par la formule générale (I) 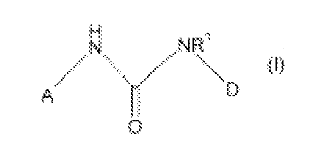 ou sel pharmaceutiquement acceptable de celui-ci, dans lequel
A représente un système cyclique choisi dans le groupe constitué par :
pyridyle, thiényle, thiazolyle, indolyle, pyrazolyle et oxo-pyrrolidinyle ;
lequel système cyclique est éventuellement substitué par un ou plusieurs substituants choisis indépendamment dans le groupe constitué par :
halogéno, trifluorométhyle, nitro, alkyle, alcoxy et phényle ; et
R¹ représente -H ; et
D représente 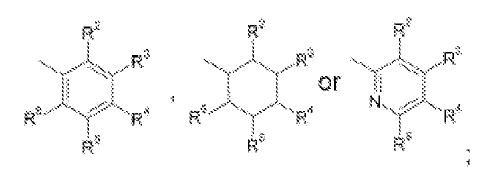 dans laquelle l'un de R², R³ et R⁴ est un tétrazolyle ;
et R⁵, R⁶ et un ou deux des R², R³ et R⁴ restants représentent indépendamment :
o hydrogène, halogéno, trifluorométhyle,
o -CH=CH-COOR^{b} ; -CH₂-CH₂-COOR^{b},
o -CO-NR^{b}-CH₂-COOR^{c} ; -CO-NR^{b}R^{c},
o -CH=CH-CO-NR^{b}R^{c} ; -CH₂-CH₂-CO-NR_{b}R^{c},
o pipéridylcarbonyle,
o -NH-CO-R^{d} ou -NH-CO-NH-R^{d} ;
où R^{d} est un phényle éventuellement substitué par un ou plusieurs substituants choisis indépendamment parmi halogéno ou trifluorométhyle ; ou
o un phényle éventuellement substitué par
-SO₂-NR^{b}R^{c}, -CO-NR^{b}R^{c}, -CO-NR^{b}-CH₂-COOR^{c} ou un pipéridylcarbonyle ;
où R^{b} et R^{c} sont indépendamment un hydrogène ou un alkyle.

2. Composé selon la revendication 1, dans lequel
A représente un pyridyle ;
lequel pyridyle est éventuellement substitué par un ou plusieurs substituants choisis indépendamment dans le groupe constitué par :
halogéno, trifluorométhyle, nitro, alkyle et alcoxy ; et
R¹ représente H ; et
D représente 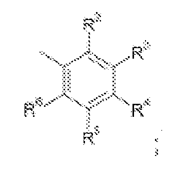 dans lequel
R² représente un tétrazolyle ;
R³, R⁴, R⁵ et R⁶ représentent indépendamment :
o un hydrogène, un halogéno, un trifluorométhyle ; ou
o un phényle substitué par
-SO₂-NR^{b}R^{c}, -CO-NR^{b}R^{c}, -CO-NR^{b}-CH₂-COOR^{c} ou un pipéridylcarbonyle ;
où R^{b} et R^{c} sont indépendamment un hydrogène ou un alkyle.

3. Composé selon la revendication 1, qui est le
*N*-(2,6-Dichloro-pyridin-4-yl)-*N*'-[3-chloro-6-(1*H-*tétrazol-5-yl)-phényl] urée ;
*N*-(2,6-Dichloro-pyridin-4-yl)-*N*'-[4'-(*N''*,*N*''-diméthyl-1-carbonyl)-2-(1*H*-tétrazol-5-yl)-biphényl-4-yl] urée ;
*N*-(2,6-Dichloro-pyridin-4-yl)-*N*'-[4-bromo-2-(1*H-*tétrazol-5-yl)-phényl] urée ;
*N*-[5-Chloro-2-(1*H*-tétrazol-5-yl)-phényl]-*N*'-(pyridin-3-yl) urée ;
*N*-[4-Bromo-2-(1*H*-tétrazol-5-yl)-phényl]-*N*'-(pyridin-3-yl) urée ;
*N*-[2,4-Dibromo-6-(1*H*-tétrazol-5-yl)-phényl]-*N*'-(2,6-dichloro-pyridin-4-yl) urée ;
ou sel pharmaceutiquement acceptable de ceux-ci.

4. Composition pharmaceutique comprenant une quantité thérapeutiquement efficace d'un composé selon l'une quelconque des revendications 1 à 3, ou d'un sel pharmaceutiquement acceptable de celui-ci, conjointement avec au moins un support, excipient ou diluant pharmaceutiquement acceptable.

5. Utilisation d'un composé selon l'une quelconque des revendications 1 à 3, ou d'un sel pharmaceutiquement acceptable de celui-ci, pour la fabrication d'une composition pharmaceutique pour le traitement, la prévention ou le soulagement d'une maladie, d'un trouble ou d'une condition chez un mammifère, y compris l'homme, laquelle maladie, trouble ou condition est sensible au blocage des canaux chlorure.

6. Utilisation selon la revendication 5, dans laquelle la maladie, le trouble ou la condition sensible au blocage des canaux chlorure est une maladie métabolique osseuse ou une maladie osseuse liée à l'ostéoclaste.

7. Utilisation selon la revendication 5, dans laquelle la maladie, le trouble ou la condition sensible au blocage des canaux chlorure est l'ostéoporose, l'ostéoporose post-ménopausique, l'ostéoporose secondaire, la métastase osseuse du cancer du sein ostéolytique, l'invasion du cancer ostéolytique, la maladie osseuse de Paget.

8. Utilisation selon la revendication 5, dans laquelle la maladie, le trouble ou la condition sensible au blocage des canaux chlorure est une maladie, un trouble ou une condition sensible à l'activité du mastocyte ou du basophile, et à l'inhibition de l'angiogenèse.

9. Utilisation selon la revendication 5, dans laquelle la maladie, le trouble ou la condition sensible au blocage des canaux chlorure est l'aspergillose broncho-pulmonaire allergique (ABPA), la rhinite allergique, la maladie cutanée allergique, la réaction cutanée allergique, la réaction cutanée allergique d'origine médicamenteuse, l'anaphylaxie, l'asthme, l'athérosclérose, la dermite atopique (DA), l'asthme bronchique, le cancer, la maladie pulmonaire obstructive chronique (MPOC), la maladie de Crohn, la dermite de contact, la myocardiopathie dilatée, l'asthme mortel, le rejet de greffe, l'alvéolite allergique extrinsèque, la cardiopathie ischémique, la fibrose pulmonaire, la polyarthrite rhumatoïde, la sclérodermie généralisée, l'urticaire, l'uvéorétinite, le cancer, le cancer métastatique, le cancer de la prostate, le cancer du poumon, le cancer du sein, le cancer de la vessie, le cancer du rein, le cancer du côlon, le cancer de l'estomac, le cancer du pancréas, le cancer de l'ovaire, le mélanome, l'hépatome, le sarcome, le lymphome, la dégénérescence maculaire exsudative, la dégénérescence maculaire liée à l'âge (DMA), la rétinopathie, la rétinopathie diabétique, la rétinopathie diabétique proliférative, la rétinopathie ischémique (par exemple occlusion de la veine ou de l'artère rétinienne), la rétinopathie de prématurité, la glaucome néovasculaire, le néovascularisation cornéenne, la polyarthrite rhumatoïde, le psoriasis, la dépranocytose, l'oedème cérébral consécutif à une ischémie ou des tumeurs, la diarrhée, l'hypertension, l'hypertension diurétique, le glaucome ou les ulcères.
